# EUROPÄISCHE PATENTANMELDUNG

(11) **EP 1 475 127 A1**
(43) Veröffentlichungstag der Anmeldung: **10.11.2004**
(21) Anmeldenummer: 03016516.1
(22) Anmeldetag: 22.07.2003
(51) Int. Cl.: A61N 5/10

(54) **Verfahren zur Dosierungsbestimmung bei der Planung von Strahlentherapie- bzw. Radiochirurgiemassnahmen**

(30) Priorität: 08.05.2003 DE 10320611
(71) Anmelder: BrainLAB AG, 85551 Kirchheim/Heimstetten (DE)
(72) Erfinder: Fröhlich, Stephan, 85609 Aschheim (DE); Nowak, Werner, 81927 München (DE); Promberger, Claus, 81673 München (DE)
(74) Vertreter: Schwabe - Sandmair - Marx

(57) **Zusammenfassung**

Die Erfindung betrifft ein Verfahren zur Dosierungsbestimmung bei der Planung von Strahlentherapie- bzw. Radiochirurgiemaßnahmen, mit den folgenden Schritten:
- das Bestrahlungszielgebiet wird mittels eines Bildgebungsverfahrens, das funktionelle bzw. biologisch aktive Bereiche des Zielgebiets unterscheiden kann, abgebildet,
- einzelnen Regionen des abgebildeten Bestrahlungszielgebiets werden ermittelte Aktivitätswerte zugewiesen,
- den Regionen werden entsprechend den Aktivitätswerten Bestrahlungsdosen zugeordnet, und
- die Solldosisverteilung, ermittelt aus den Bestrahlungsdosen für die Regionen, wird als Eingangswert für die Behandlungsplanung verwendet.

## Beschreibung

Die Erfindung betrifft ein Verfahren zur Dosierungsbestimmung bei der Planung von Strahlentherapie- bzw. Radiochirurgiemaßnahmen. Die Bestrahlung bestimmter Zielgebiete von Patienten, beispielsweise die Bestrahlung von Tumoren wird nach heutigem Stand der Technik computerunterstützt geplant und erfolgt dann auf der Basis dieser Planung durch computergesteuerte Bestrahlungsgeräte. In der Regel werden hier Bildgebungsverfahren wie die Computertomographie oder die Kemspintomographie zu Hilfe genommen, um die Aussenkonturen der zu bestrahlenden Region zu bestimmen, wobei eine solche Aussenkontur meist in die erhaltenen Schichtbilder eingezeichnet wird. Ein so bestimmtes Bestrahlungszielgebiet wird gemäß der herkömmlichen Bestrahlungstechnik in der Regel möglichst homogen bestrahlt, wobei es grundsätzlich keine Rolle spielt, ob die vorab erfolgte Planung "invers" oder konventionell erfolgt.

Bei der konventionellen Planung wird einfach ein bestimmtes Bestrahlungsziel ausgewählt und festgelegt, mit welcher Dosis dieses Gebiet bestrahlt werden soll. Die Bestrahlung erfolgt dann dementsprechend.

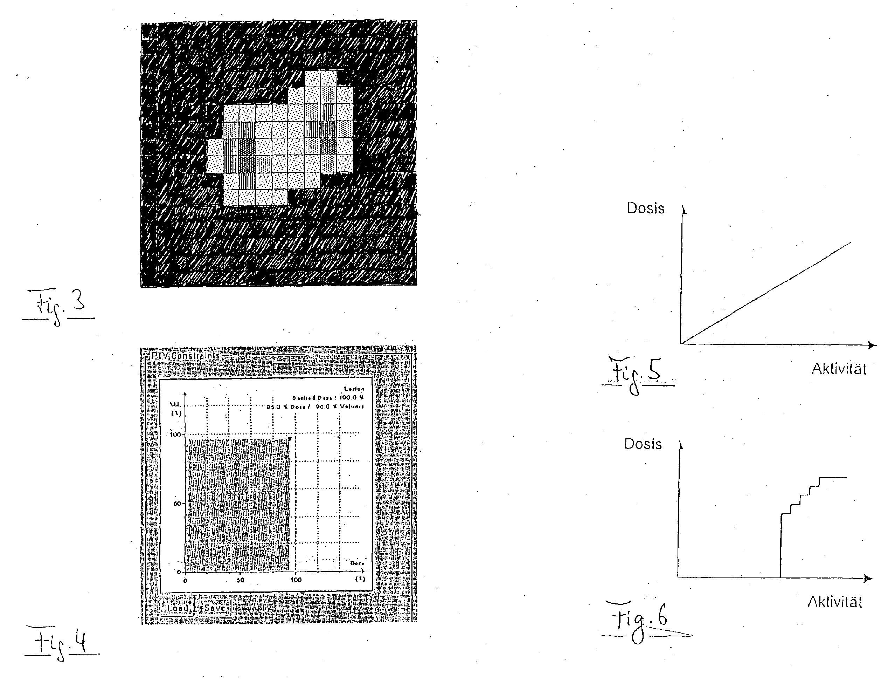

Die Dosisbestimmung bzw. Dosisverschreibung für die inverse Bestrahlungsplanung erfolgt anders. Es werden in der Regel Histogramme (Dosis-Volumen-Histogramme) verwendet, wie eines beispielsweise in der anliegenden Figur 4 gezeigt ist. Da die perfekte Homogenität technisch meistens nicht erreicht werden kann, ohne Risikostrukturen zu schädigen, folgt die Verschreibung beispielsweise folgendem Ansatz:
- 80% des Tumorvolumens sollen mit mindestens 90% der vorgeschriebenen Dosis bestrahlt werden;
- 95% des Tumorvolumens sollen mit mindestens 60% der verschriebenen Dosis bestrahlt werden; usw.

Das Problem solcher herkömmlicher Bestrahlungsplanungen liegt darin, dass das Bestrahlungszielgebiet als homogene Masse angesehen wird, obwohl dies insbesondere bei Tumoren nicht der Fall ist. Tumoren weisen Regionen mit höherer Aktivität bzw. Aggressivität auf, und andererseits auch Regionen mit niedriger Aktivität bzw. Aggressivität. Das Vorhandensein solcher unterschiedlicher Regionen wird bei der herkömmlichen Bestrahlungsplanung, ob konventionell oder invers in der Regel nicht berücksichtigt, was dazu führt, dass bestimmte Regionen mehr oder weniger Strahlung erhalten als dies für einen optimalen Therapieerfolg notwendig wäre.

Es ist die Aufgabe der vorliegenden Erfindung, ein Verfahren zur Dosierungsbestimmung bei der Planung von Strahlentherapie- bzw. Radiochirurgiemassnahmen vorzuschlagen, das die oben aufgeführten Probleme des Standes der Technik überwindet. Insbesondere soll eine optimierte Dosierungsplanung im Hinblick auf inhomogene Aktivitäten bzw. Aggressivitäten von Behandlungszielen ermöglicht werden.

Diese Aufgabe wird durch ein Verfahren gelöst, wie es im Patentanspruch 1 beschrieben wird. Die Unteransprüche definieren vorteilhafte Ausführungsformen der Erfindung.

Die Erfindung wird nunmehr, unter anderem anhand der beiliegenden Figuren näher erläutert. Es zeigen:
Figur 1 eine funktionelle Schichtbilddarstellung des menschlichen Kopfes mit einem sichtbaren Tumor;
Figur 2 eine Ausschnittvergrößerung des in Figur 1 aufgezeigten Schichtbildes;
Figur 3 eine Clasterung eines nuklearmedizinischen Bilddatensatzes in Bereiche definierter Aktivität;
Figur 4 ein Dosis-Volumen-Histogramm für die inverse Planung;
Figur 5 und 6 Diagramme für einen linearen bzw. nicht linearen Zusammenhang zwischen Aktivität und Verschreibungsdosis.

Die Vorteile der vorliegenden Erfindung beruhen insbesondere darauf, dass Informationen aus Bildgebungsverfahren verwertet werden, die funktionelle bzw. biologisch aktive Bereiche des Bestrahlungszielgebietes unterscheiden können. Informationen über diese "aktiven" oder "weniger aktiven" Regionen können verwendet werden, um eine solche Aktivitäts-Inhomogenität auch bei der Bestrahlungsplanung zu berücksichtigen.

Bisher bei der Bestrahlungsplanung verwendete Bildgebungsverfahren wie Computertomographie, Kernspintomographie und Röntgen bieten mehr oder weniger lediglich eine geometrische Abbildung interner Strukturen. Andere Verfahren, aber möglicherweise auch weiter entwickelte Verfahren, wie sie oben beschrieben wurden, gewinnen immer mehr an Bedeutung, beispielsweise nuklearmedizinische Verfahren wie PET und SPECT. Diese bildgebenden Verfahren können biologische Aspekte darstellen. Solche biologischen Aspekte können beispielsweise Stoffwechselaktivität, Durchlässigkeit der Zellwand für bestimmte Stoffe usw. sein. In der vorliegenden Beschreibung sind sie als solche gekennzeichnet, die funktionelle bzw. biologisch aktive Bereiche unterscheiden können. Aus diesen Informationen können auch die Aktivität bzw. die Aggressivität in verschiedenen Regionen eines Tumors erfasst werden.

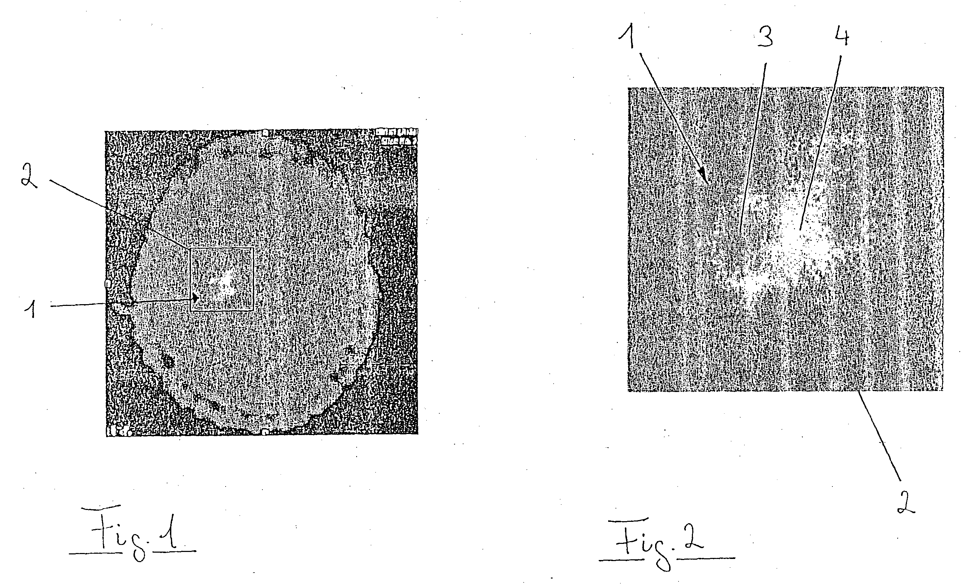

Die Figur 1 zeigt eine nuklearmedizinische Schichtbilddarstellung des menschlichen Kopfes mit einem Tumor, der das Bezugszeichen 1 trägt. Es handelt sich hierbei um eine PET-Darstellung. In Figur 1 ist mit einem Quadrat ein Ausschnitt 2 aufgezeigt, der vergrößert in Figur 2 dargestellt ist.

In der Ausschnittsvergrößerung der Figur 2 lassen sich Regionen unterschiedlicher Aktivität im Tumor 1 erkennen. Eine dunklere Stelle ist mit dem Bezugszeichen 3 angedeutet, und sie bezeichnet einen Bereich niedriger Aktivität bzw. niedrigerer Aggressivität des Tumors. Einen hellen Bereich bezeichnet das Bezugszeichen 4, und dort ist die Aktivität bzw. Aggressivität höher.

Mit der vorliegenden Erfindung wird es nunmehr möglich, von der bisherigen, möglichst homogenen Bestrahlung abzugehen und Tumoren inhomogen, abhängig von der Aktivität der jeweiligen Region zu bestrahlen. Aktive oder "heiße" Regionen werden stark bestrahlt, "lauwarme" schwächer.

Erfindungsgemäß werden den Regionen entsprechend den ermittelten Aktivitätswerten Bestrahlungsdosen zugeordnet. Mit anderen Worten wird die Aktivität einer Tumor-Region direkt oder indirekt in die vorgeschriebene Dosis für eine entsprechende Tumorregion transferiert.

Einerseits kann hierbei konventionell geplant werden, jedoch ist die Erfindung besonders vorteilhaft für die inverse Planung. "Inverse Planung" bedeutet hier ganz allgemein, dass zunächst vorgeschrieben wird, welcher Anteil des Volumen welche Bestrahlungsdosis erhalten soll.

Die Erfindung gestattet es nun, eine solche inverse Planung, die den Tumor als homogene Einheit ansah, insoweit zu verbessern, dass noch zusätzlich berücksichtigt wird, wo im Tumor die aktiveren bzw. die weniger aktiven Regionen sind.

Die Aktivität der Tumorregion kann direkt in linearem Zusammenhang in die vorgeschriebene Dosis für die entsprechende Tumorregion transferiert werden. Eine solche direkte Abhängigkeit zwischen Dosis und Aktivität ist beispielsweise in der Figur 5 gezeigt. Die Figur 6 zeigt eine andere Zuordnung, bei der die Aktivität in nicht linearer bzw. vom Anwender definierter Abhängigkeit in die Dosisverschreibung transferiert wird. Ein nicht linearer Zusammenhang kann vorab festgelegt werden, es kann aber auch manuell eine Definition des Zusammenhangs erfolgen.

Mit der Erfindung ergibt sich ein zeitsparender Weg zur inhomogenen, biologischen Dosisverschreibung. Die Dosisverschreibung erfolgt ortsgenau; bei inverser Planung wird auf jede einzelne Region, nicht mehr auf das komplette Zielvolumen abgezielt. Im klinischen Bereich ermöglicht die vorliegende Erfindung eine schonendere Bestrahlung z.B. dann, wenn eine Region des Tumors mit geringerer Aktivität nahe an einer kritischen (= strahlen sensiblen) Struktur liegt, die eigentlich keine Bestrahlung benötigt. Außerdem ist eine Maximierung der Dosis in besonders aktiven Tumorregionen möglich.

Gemäß der Erfindung können Regionen ähnlicher Aktivität zusammengefasst werden, so dass eine begrenzte Anzahl diskreter Aktivitätslevels entsteht. Die Figur 3 zeigt hierzu ein Beispiel für eine Einteilung eines nuklearmedizinischen Bilddatensatzes in definierte Areale, wobei eine definierte Dosis pro Areal zugewiesen wird. Die dunkleren Stellen in Figur 3 bedeuten eine niedere Dosis, die helleren Grauabstufungen gehen hin zu höheren Dosierungen. Die Figur 3 ist eine flächenmäßige Darstellung dieser Regionen; es ist jedoch ohne weiteres auch möglich, eine solche Darstellung dreidimensional anzuzeigen und bei der Planung zu verwerten.

Im Rahmen der Erfindung ist es auch möglich, eine Bestrahlungsdosis nur bestimmten Benutzer definierten Bereichen zuzuordnen, während die restlichen Bereiche des Bestrahlungszielgebietes keine Bestrahlungsdosis erhalten.

Es besteht ferner die Möglichkeit, auch die oben angesprochenen konventionelleren Bildgebungsverfahren wie Computertomographie, Kemspintomographie oder Röntgen, die in ihrer Basisausführung keine funktionell bzw. biologisch aktiven Bereiche unterscheiden können, noch hilfsweise hinzuzuziehen. Möglicherweise liefern diese Bilderzeugungsverfahren nämlich noch zusätzliche Informationen bzw. gestatten eine bessere Visualisierung des Behandlungszielgebietes. Wenn die Bilder aus den verschiedenen Aufnahmetechniken zueinander registriert werden, d.h. durch eine Navigation bzw. ein Tracking der Aufnahme sichergestellt wird, das beide Bilder denselben Schnitt zeigen, ist es möglich, die Informationen aus den verschiedenen Aufnahmeverfahren optimal zu kombinieren und zu verwerten.

Die Bestrahlungsdosis kann einerseits der jeweiligen Region entsprechend dem Aktivitätswert als absoluter Wert zugeordnet werden, beispielsweise 1,25 Gy. Andererseits ist es aber auch möglich, die Bestrahlungsdosis als relativen Wert zuzuordnen, beispielsweise als 80 % der Normierungsdosis. Die Verschreibungsdosis bzw. die Bestrahlungsdosis für die Planung kann sich auf das zu bestrahlende Zielvolumen (die Zielvolumina) beziehen. Es gibt auch bildgebende Verfahren, die beispielsweise die Aktivität einer bestimmen Hirnregion bei einer bestimmten Tätigkeit anzeigen (z.B. das Sprachzentrum beim Sprechen). Wenn dies berücksichtigt wird, kann die Bestrahlungsdosis sich auf strahlensensible Strukturen beziehen, welche möglichst nur gering bestrahlt werden dürfen. Es ist natürlich auch möglich, die Bestrahlungsdosis bzw. Verschreibungsdosis sowohl auf das zu bestrahlende Zielvolumen als auch auf strahlensensible Strukturen zu beziehen.

## Patentansprüche

1. Verfahren zur Dosierungsbestimmung bei der Planung von Strahlentherapie- bzw. Radiochirurgiemaßnahmen, mit den folgenden Schritten:
- das Bestrahlungszielgebiet wird mittels eines Bildgebungsverfahrens, das funktionelle bzw. biologisch aktive Bereiche des Zielgebiets unterscheiden kann, abgebildet,
- einzelnen Regionen des abgebildeten Bestrahlungszielgebiets werden ermittelte Aktivitätswerte zugewiesen,
- den Regionen werden entsprechend den Aktivitätswerten Bestrahlungsdosen zugeordnet, und
- die Solldosisverteilung, ermittelt aus den Bestrahlungsdosen für die Regionen, wird als Eingangswert für die Behandlungsplanung verwendet.

2. Verfahren nach Anspruch 1, bei dem die Solldosisverteilung als Eingangswert für eine inverse Behandlungsplanung verwendet wird.

3. Verfahren nach Anspruch 1 oder 2, bei dem die einzelnen Regionen solche von definierter Größe und insbesondere dreidimensionale Regionen sind.

4. Verfahren nach einem der Ansprüche 1 bis 3, bei dem ein linearer Zusammenhang zwischen den Aktivitätswerten und den zugeordneten Bestrahlungsdosen verwendet wird.

5. Verfahren nach einem der Ansprüche 1 bis 3, bei dem ein nicht linearer Zusammenhang zwischen den Aktivitätswerten und den zugeordneten Bestrahlungsdosen verwendet wird.

6. Verfahren nach einem der Anspruch 5, bei dem der nicht lineare Zusammenhang zwischen den Aktivitätswerten und den zugeordneten Bestrahlungsdosen manuell definiert wird.

7. Verfahren nach einem der Ansprüche 1 bis 6, bei dem Regionen ähnlicher Aktivität zusammengefasst werden, so dass eine begrenzte Anzahl diskreter Aktivitätslevels existiert.

8. Verfahren nach einem der Ansprüche 1 bis 7, bei dem eine Bestrahlungsdosis nur bestimmten benutzerdefinierten Bereichen zugeordnet wird, während die restlichen Bereiche des Bestrahlungszielgebiets keine Bestrahlungsdosis erhalten sollen.

9. Verfahren nach Anspruch 8, bei dem die Bereiche, denen eine Bestrahlungsdosis zugeordnet wird, mit Hilfe von Bildinformationen ausgewählt werden, die von Bildgebungsverfahren stammen, die funktionelle bzw. biologisch aktive Bereiche des Zielgebiets nicht unterscheiden können, wobei die Bildinformationen aus beiden Bildgebungsverfahren zueinander registriert werden.

10. Verfahren nach einem der Ansprüche 1 bis 9, bei dem die Bestrahlungsdosis als absoluter Wert zugeordnet wird.

11. Verfahren nach einem der Ansprüche 1 bis 9, bei dem die Bestrahlungsdosis als relativer Wert zugeordnet wird, insbesondere als ein Prozentsatz einer Normierungsdosis.

12. Verfahren nach einen der Ansprüche 1 bis 11, bei dem die Bestrahlungsdosis zugeordnet wird unter Bezugnahme auf
- das Bestrahlungsziel, nämlich Bereiche mit gewünschter hoher Bestrahlung,
- Bereiche mit gewünschter niedriger Bestrahlung,
- oder unter Bezugnahme auf beide genannten Bereiche.
